(19) Europäisches Patentamt
European Patent Office
Office européen des brevets

(11) Veröffentlichungsnummer: **0 324 191**
**A1**

(12) # EUROPÄISCHE PATENTANMELDUNG

(21) Anmeldenummer: **88121928.1**

(22) Anmeldetag: **31.12.88**

(51) Int. Cl.⁴: **C07C 43/23 , C07C 41/30 , //C07C49/255**

(30) Priorität: **08.01.88 DE 3800306**

(43) Veröffentlichungstag der Anmeldung:
**19.07.89 Patentblatt 89/29**

(84) Benannte Vertragsstaaten:
**BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Smuda, Hubert, Dr.**
**Bierhelderweg 7**
**D-6900 Heidelberg(DE)**
Erfinder: **Rohr, Wolfgang, Dr.**
**In der Dreispitz 13**
**D-6706 Wachenheim(DE)**

(54) **Tert.-Butylalkinole.**

(57) Neue tert.-Butylalkinole der Formel (I)

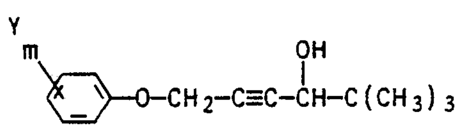

(I)

in welcher
Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht
m eine ganze Zahl von 1 bis 3 ist,
wobei die einzelnen Gruppen Y gleich oder verschieden sein können, wenn m > 1 ist und ein Verfahren zu ihrer Herstellung.

EP 0 324 191 A1

## Tert.-Butylalkinole

Die vorliegende Erfindung betrifft neue tert.-Butylalkinole der Formel (I)

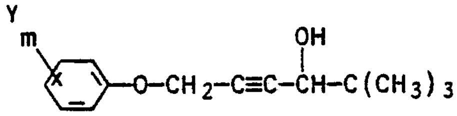 (I)

in welcher
Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht und
m eine ganze Zahl von 1 bis 3 ist,
wobei die einzelnen Gruppen Y gleich oder verschieden sein können, wenn m > 1 ist sowie ein Verfahren zu deren Herstellung.

Die erfindungsgemäßen tert.-Butylalkinole sind Ausgangsstoffe zur Herstellung von 1,2,4-Triazolyl-substituierten Fungiziden, wie sie in DE-A 30 19 049 und EP-B 150 404 beschrieben sind. Deren Herstellung gelang bislang jedoch noch nicht in zufriedenstellender Weise.

So sind diese Verbindungen nach den bisher bekannten Methoden (s. DE-A 30 19 049, EP-B 150 404) nur in unbefriedigenden Ausbeuten oder nur mit großem präparativen Aufwand herstellbar. Diese Verfahren sind somit zur industriellen Nutzung aus Gründen der Wirtschaftlichkeit wenig geeignet. Der Erfindung lag daher die Aufgabe zugrunde, diese Wirkstoffe durch Bereitstellung neuer Ausgangsstoffe besser zugänglich zu machen.

Dementsprechend wurden tert.-Butylalkinole der allgemeinen Formel (I) gefunden,

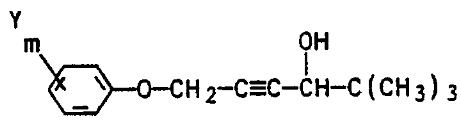 (I)

in welcher
Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht
m eine ganze Zahl von 1 bis 3 ist,
wobei die einzelnen Gruppen Y gleich oder verschieden sein können, wenn m > 1 ist.

Außerdem wurde ein Verfahren zur Herstellung von tert.-Butylalkinole der Formel (I)

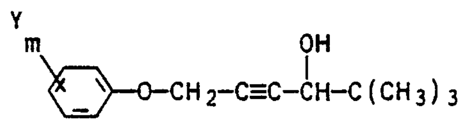 (I)

in welcher
Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht
m eine ganze Zahl von 1 bis 3 ist,
wobei die einzelnen Gruppen Y gleich oder verschieden sein können, wenn m > 1 ist, gefunden, das dadurch gekennzeichnet ist, daß man Phenylpropargylether der Formel (II)

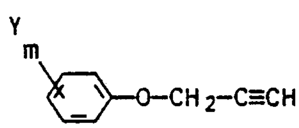 (II)

mit 2,2-Dimethylpropanal in Gegenwart einer Mineralbase in einem polar-aprotischen Lösungsmittel zu den tert.-Butylalkinolen (I) umsetzt.

Der Phenoxyrest ist bevorzugt folgendermaßen durch $Y_m$ substituiert:
Wasserstoff, 2-Fluor, 3-Fluor, 4-Fluor, 2-Chlor, 3-Chlor, 4-Chlor, 4-Brom, 2,4-Dichlor, 2,4,6-Trichlor, 3,4-Dichlor, 3,5-Dichlor, 2-Chlor-4-phenyl, 2-Methyl-4-chlor, 2-Methyl, 3-Methyl, 4-Methyl, 3-tert.-Butyl, 4-tert.-Butyl, 2-Methoxy, 3-Methoxy, 4-Methoxy, 3,5-Dimethoxy, 3-n-Butoxy, 4-n-Butoxy, 2-Methoxy-4-methyl, 3-Trifluormethyl, 4-Trifluormethyl, 4-Phenoxy.

Besonders bevorzugt steht m für 1 und Y für Wasserstoff, 4-Methoxy, 2-Chlor, 3-Chlor oder 4-Chlor, 4-tert.-Butyl, 2-Methyl oder 3-Methyl.

Die Herstellung der erfindungsgemäßen tert.-Butylalkinole I erfolgt vorteilhaft in der Weise, daß man Phenylpropargylether der Formel (II)

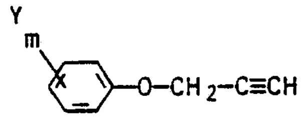

(II),

mit 2,2-Dimethylpropanal in Gegenwart einer für Favorskii-Reaktionen geeigneten Base in einem polar-aprotischen Lösungsmittel zu den tert.-Butylalkinolen (I) umsetzt.

Die als Ausgangsstoffe verwendeten Phenylpropargylether (II) sind an sich bekannt (vgl. z.B. die niederländischen Anmeldungen 6 614 925 vom 24.04.1967 oder 6 612 645 vom 09.03.1966) oder können nach bekannten Methoden hergestellt werden (z.B. nach Pourcelot, Cadiot: Bull. Soc. Chim. Fr. 1966, 3016, 3024; McKillop et al: Tetrahedron 30, 1379, (1974)), beispielsweise indem man ein ggf. substituiertes Phenol mit einem Propargylderivat $XCH_2C\equiv CH$, in dem X für eine Abgangsgruppe steht, in Gegenwart einer Base gemäß folgender Reaktionsgleichung umsetzt:

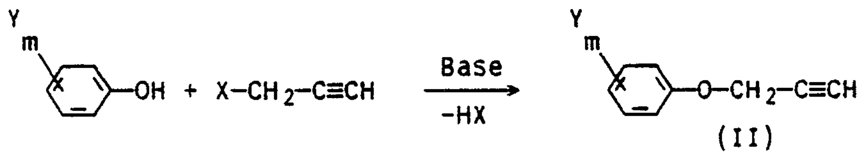


(II)

Die Umsetzung der Phenylpropargylether (II) mit 2,2-Dimethylpropanal verläuft im Sinne einer Favorskii-Reaktion (vgl. Merck Index, 9th edition, ONR-29, 1976 und Houben-Weyl, Methoden der organischen Chemie, Bd. V/2a, 509-519, 1977 und dort zitierte Literatur) gemäß der folgenden Reaktionsgleichung:

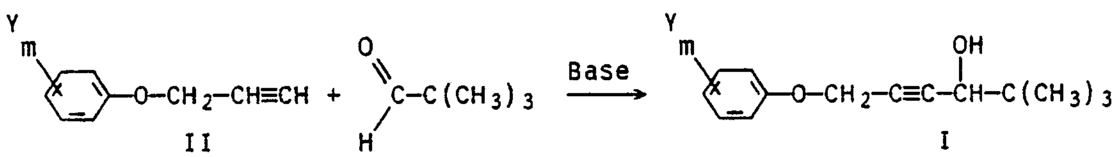


II I

Geeignete Basen zur Durchführung der Umsetzung sind z.B. Grignard-Reagentien, Organo-Lithiumverbindungen oder Alkalimetallhydroxide, insbesondere Kaliumhydroxid. Wegen der gegenüber Grignard-Reagentien oder Organo-Lithiumverbindungen erheblich geringeren Hydrolyseempfindlichkeit wird Kaliumhydroxid besonders bevorzugt verwendet. Ferner hat Kaliumhydroxid als Base den Vorteil, bereits in katalytischen Mengen wirksam zu sein.

Die Menge des bei der Umsetzung verwendeten Kaliumhydroxids ist nicht kritisch. Dementsprechend kann das Kaliumhydroxid im Überschuß, in äquivalenter oder katalytischer Menge angewandt werden. Bevorzugt wird das Kaliumhydroxid in Mengen von 0,01 bis 1,0, insbesondere von 0,5 bis 1,0 Äquivalenten, bezogen auf den umzusetzenden Propargylether, verwendet.

Pro Mol 2,2-Dimethylpropanol können 0,5 - 1,0 mol Propargylether eingesetzt werden, jedoch sind äquimolare Mengen bevorzugt.

Die Umsetzung kann in üblichen polar-aprotischen organischen Lösungsmitteln vorgenommen werden. Bevorzugt werden Ether, z.B. Diethylether, Glykoldimethylether, Triglykoldimethylether, Tetrahydropyran

und insbesondere Tetrahydrofuran. Die Reaktionstemperatur kann 0 bis 80, vorzugsweise 20 bis 60 °C betragen.

Die Aufarbeitung erfolgt vorteilhaft in der Weise, daß man die Reaktionsmischung neutralisiert und mit Wasser und einem wasserunmischbaren organischen Lösungsmittel, z.B. Toluol, extrahiert. Nach Eindampfen der Extraktionslösungen können die Rohprodukte durch geeignete Verfahren wie Säulenchromatographie weiter gereinigt werden.

Die erfindungsgemäßen tert.-Butylalkinole (I) dienen als Zwischenprodukte zur Herstellung von Azolylwirkstoffen wie den in der DE-A 30 19 049 beschriebenen 1,2,4-Triazolyl-1-derivaten.

Zu diesem Zweck werden die erfindungsgemäßen tert.-Butylalkinole (I) durch Oxidation mit gängigen Oxidationsmitteln, beispielsweise Natriumdichromat/Schwefelsäure, Mangandioxid oder Nickelperoxid, oder elektrochemisch, durch anodische Oxidation, in die Phenoxyalkinylketone (IV) überführt. Die anschließende katalytische Hydrierung der Dreifachbindung, beispielsweise mit Palladiumkatalysatoren in Methanol, liefert dann die Phenoxyalkylketone (III).

Diese Reaktionssequenz ist in folgendem Schema dargestellt:

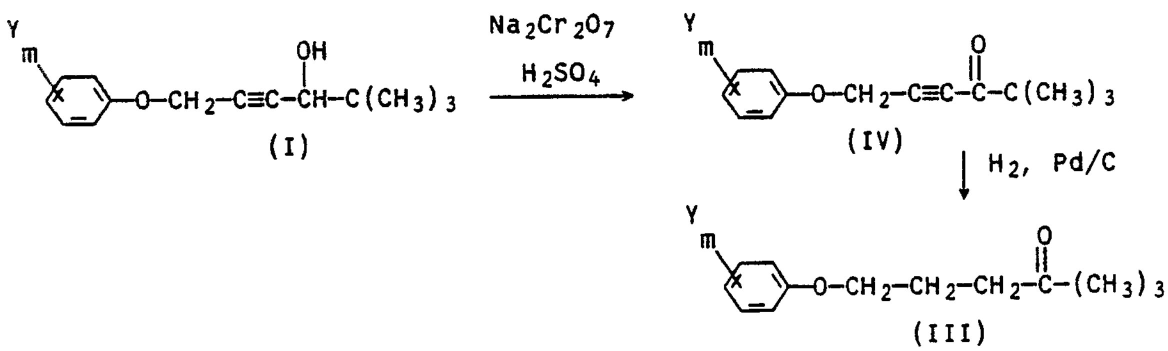


(I) (IV) (III)

Aus den Phenoxyalkylketonen (III) können in bekannter Weise, wie in der EP-B 150 404 beschrieben, die Azolylwirkstoffe synthetisiert werden.

Beispiele

Die zur Herstellung der tert.-Butylalkinole der Beispiele 1 bis 12 benötigten Propargylether (II) wurden nach den Verfahren von Pourcelot et al (Bull. Soc. Chim. Fr. 1966, 3016; 3024) und McKillop et al (Tetrahedron 30, 1379 (1974)) hergestellt.

Allgemeine Vorschrift zur Herstellung der tert.-Butylalkinole

Ein Äquivalent eines Phenylpropargylethers II wurde in dem 5-fachen Volumen Tetrahydrofuran vorgelegt. Es wurde ein Äquivalent festes Kaliumhydroxid zugesetzt und anschließend 1,2 Äquivalente Pivalaldehyd (2,2-Dimethylpropanal) zugetropft. Nachdem die gesamte Menge Pivalaldehyd zugesetzt war, wurde bei 50 °C gerührt, bis eine HPLC-Analyse (Hochdruckflüssigkeitschromatographie) einer entnommenen Probe vollständigen Verbrauch des Phenylpropargylethers anzeigte.

Zur Aufarbeitung wurde von ungelöstem Kaliumhydroxid abfiltriert, dem Filtrat 5 Gew.-% Wasser zugesetzt und überschüssiges Alkali mit 10 gew.%iger Salzsäure neutralisiert. Der Mischung wurde Toluol zugesetzt, die Wasserphase abgetrennt und die Toluolphase mit Wasser extrahiert. Nach Trocknen der Toluolphase über Natriumsulfat wurden die organischen Lösungsmittel abgedampft. Die tert.-Butylalkinole erhielt man als gelbe Öle, die sich beim Destillationsversuch zersetzten.

Nach dieser Methode wurden die in folgender Tabelle 1 aufgeführten tert.-Butylalkinole erhalten.

Tabelle 1

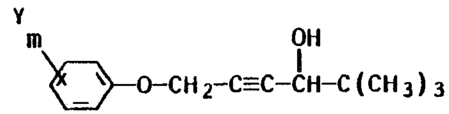

I

| Bei-spiel | $Y_m$ | Ausbeute % der Theorie | $^1H$-NMR ($CDCl_3$) a)<br>$^{13}C$-NMR ($CDCl_3$) a) |
|---|---|---|---|
| 1 | H | 92 | δ = 7.32(m;2H); 7.0(m;3H); 4.78(s;2H); 4.05(d;1H); 1,8(d;OH); 0,92(s;9H)<br>δ = 157, 129 (2xC) 121, 115 (2xC), 87, 80, 71, 56, 36, 25 (3xC) |
| 2 | Cl | 69 | δ = 7.1(d;2H); 6.95(d;2H); 4.75(s;2H); 4.05(s;1H); 2.4(s;OH); 0.95(s;9H) |
| 3 | 4-$C(CH_3)_3$ | 54 | δ = 7.3(d;2H); 6.9(d;2H); 4.75(s;2H); 4.05(s;1H); 1.95(s verbreitert; OH); 1.25(s;9H); 0.95(s;9H);<br>δ = 155.4; 144.1; 129.0 (2xC) 114.7 (2xC), 87.2; 80.7; 71.2; 56.2; 35.8; 34.1; 31.5 (3xC); 29.7 (3xC) |
| 4 | 2-$CH_3$ | 78 | δ = 7.1(m;2H); 6.85(m;2H); 4.7(s;2H); 4.0(s;1H); 2.2(s;3H); 0.95(s;9H)<br>δ = 156.1; 130.9; 127.5; 126.7; 121.5; 112.6; 87.3; 81.0; 71.3; 56.5; 35.8; 25.3; 16.0 |
| 5 | 3-$CH_3$ | 77 b) | δ = 7.1(t;1H); 6.75(d;3H); 4.68(s;2H); 4.00(s;1H); 2.4(s verbreitert;OH); 2.3(s;3H); 0.95(s;9H)<br>δ = 157.9; 139.3; 129.2; 122.5; 116.3; 112.4; 112.1; 87.5; 80.9; 71.3; 56.2; 35.8; 28.8; 21.4 |
| 6 | 4-$CH_3$ | 72 | δ = 7.0(d;2H); 6.8(d;H); 4.6(s;2H); 4.0(s;1H); 2.3(S;3H); 1.0(s;9H);<br>δ = 156, 131, 130(2xC), 115(2xC), 88, 81, 71, 57, 36, 25(3xC) |

Tabelle 1 (Fortsetzung)

| Bei-spiel | $Y_m$ | Ausbeute % der Theorie | $^1H$-NMR ($CDCl_3$) (a)<br>$^{13}C$-NMR ($CDCl_3$) (a) |
|---|---|---|---|
| 7 | 4-$CH_3$ | 76 | δ = 6.8(dd;4H); 4.6(s;2H); 4.0(s;1H); 3.65(s;3H); 2,5(s verbreitert;OH); 0.95(s;3H)<br>δ = 155, 152, 117 (2xC); 115 (2xC); 88, 81, 71, 57, 56, 36, 25 (3xC) |
| 8 | 2-$OCH_3$ | 85 | δ = 6.85(m;4H); 4.7(s;2H); 3.95(s;1H); 3.8(s;3H); 0.9(s;9H)<br>δ = 151, 148, 123, 121, 116, 113, 88, 81, 71, 58, 56, 36, 25 (3xC) |
| 9 | 3-F | 77 | δ = 7.2(m;1H); 6.7(m;4H); 4.7(s;2H); 4.0(s;1H); 0.95(s;9H)<br>δ = 163.6(d;$J_{C-F}$=245,7 Hz); 159(d); 130, 111, 108, 103; 88, 80, 71, 57, 36, 25 (3xC) |
| 10 | 4-F | 93 | δ = 7.0(d;2H); 6.9(d;2H); 4.7(s;2H) 4.0(s;1H); 2.9 (s verbreitert, OH); 0.9(s;9H)<br>δ = 158 (d;$J_{C-F}$=239,1 Hz); 154, 117(2xC); 116(2xC); 88, 81, 71, 57, 36, 25(3xC) |
| 11 | 2,4-$F_2$ | 85 | δ = 7,05(m;1H); 6.8(m;2H); 4.75(s;2H); 4.0(s;1H); 0.9(s;9H)<br>δ = 157(d;$J_{C-F}$=243,7 Hz); 154 (d; $J_{C-F}$=250,4 Hz); 142, 119, 111, 105 (d,d); 89, 80, 71, 59, 36, 25(3xC) |
| 12 | 3-$CF_3$ | 91 | δ = 7,4(m;1H); 7,2(m;3H); 4,7(s;2H); 4,0(s,1H); 2,6(verbreitert, OH); 0,9(s;9H)<br>δ = 158; 132(m;$J_{C-F}$=32,8Hz); 130; 124(m;$J_{C-F}$=272,1Hz); 119, 118; 112; 88; 80; 72; 57; 36; 29(3xC). |

a) die chemische Verschiebung δ bezieht sich auf den Standard Tetramethylsilan (TMS) = 0
b) CH-Analyse: Ber.: C 77.55; H 8.68; Gef.: C 77.30; H 8.50

Beispiele für Folgestufen:

Herstellung des 2,2-Dimethyl-6-phenoxy-hexin-4-on-3:

4,4 g (0,02 mol) 2,2-Dimethyl-6-phenoxy-hexin-4-ol-3 wurden in 10 ml Aceton gelöst, wonach eine Lösung von 3 g $K_2Cr_2O_7$ und 7 g konz. $H_2SO_4$ in 10 ml Wasser zugetropft wurde. Nachdem eine HPLC-Analyse einer Probe vollständigen Umsatz anzeigte, wurde die Reaktionslösung mit Methylenchlorid extrahiert. Die organische Phase wurde abgetrennt mit Wasser gewaschen, über $Na_2SO_4$ getrocknet, filtriert und eingeengt. Man erhielt 2,6 g eines gelben Öls, entsprechend 60 % der Theorie.
$^1$H-NMR ($CDCl_3$) $\delta$ = 7.25 (m;2H); 6.9 (m;3H); 4.8 (s;2H);
$^{13}$C-NMR ($CDCl_3$) $\delta$ = 193, 157, 130 (2xC), 122, 115 (2xC), 88, 84, 56, 44, 26 (3xC)
IR: 2971, 2200, 1674, 1599, 1589, 1235, 1213, 1141, 1039, 754, $cm^{-1}$

Katalytische Hydrierung des 2,2-Dimethyl-6-phenoxy-hexin-4-on-3

2 mol rohes 2,2-Dimethyl-6-phenoxy-hexin-4-on-3 wurden in einem 4-l-Kolben, der an eine Hydrierapparatur angeschlossen war, in 1,8 l Methanol gelöst und mit 20 g Hydrierkatalysator (10 % Palladium auf Aktivkohle) versetzt. Nach Evakuieren der Apparatur wurde der Gasraum mit Wasserstoff gefüllt und unter Rühren die Hydrierung bei Raumtemperatur in Gang gebracht. Durch freiwerdende Hydrierwärme erwärmte sich die Reaktionslösung auf 35-40 °C. Nachdem 103 l (4 mol) Wasserstoff aufgenommen worden waren, kam die $H_2$-Aufnahme zum Stillstand. Wasserstoffreste wurden mit Stickstoff ausgeblasen. Nach Abfiltrieren vom Katalysator wurde eingeengt und das rohe 2,2-Dimethyl-6-phenoxy-hexanon-3 im Vakuum destilliert.
$Kp_{1,0}$: 140 °C, Ausbeute: 89 % der Theorie
$^1$H-NMR ($CDCl_3$) $\delta$ = 7,2 (m;2H); 6,9 (m;3H); 3,95 (dd;2H); 2,65 (dd;2H); 2,0 (qt;2H); 1.12 (s;9H)
$^{13}$C-NMR ($CDCl_3$): $\delta$ = 214, 159, 129 (2xC), 121, 115 (2xC), 67, 34, 26, 24.

## Ansprüche

1. Tert.-Butylalkinole der allgemeinen Formel (I)

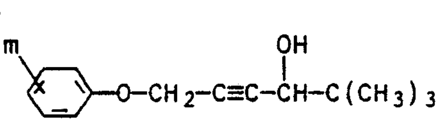

(I)

in welcher
Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht und
m eine ganze Zahl von 1 bis 3 ist,
wobei die einzelnen Gruppen Y gleich oder verschieden sein können, wenn m > 1 ist.

2. Verfahren zur Herstellung von tert.-Butylalkinolen der Formel (I)

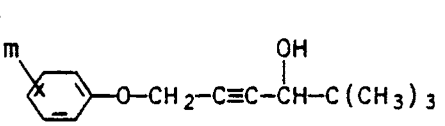

(I)

in welcher
Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht und
m eine ganze Zahl von 1 bis 3 ist,

wobei die einzelnen Gruppen Y gleich oder verschieden sein können, wenn m > 1 ist, dadurch gekennzeichnet, daß man Phenylpropargylether der Formel (II)

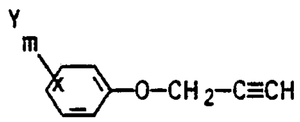

(II)

mit 2,2-Dimethylpropanal in Gegenwart einer Mineralbase in einem polar-aprotischen Lösungsmittel umsetzt.

3. Verfahren nach Anspruch 2, dadurch gekennzeichnet, daß man Kaliumhydroxid als Base verwendet.

4. Verfahren nach den Ansprüchen 2 oder 3, dadurch gekennzeichnet, daß man 0,5 bis 1 Äquivalente Kaliumhydroxid, bezogen auf (II), verwendet.

5. Verfahren nach den Ansprüchen 2 bis 4, dadurch gekennzeichnet, daß man als Lösungsmittel Ether verwendet.

Patentansprüche für folgenden Vertragsstaat: ES

1. Verfahren zur Herstellung von tert.-Butylalkinolen der Formel (I)

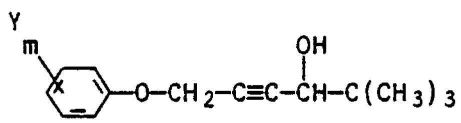

(I)

in welcher

Y für Wasserstoff, Halogen, $C_1$-$C_4$-Alkyl, $C_1$-$C_4$-Alkoxy, $C_1$-$C_4$-Halogenalkyl, Aryl, Aryloxy oder Benzyl steht und

m eine ganze Zahl von 1 bis 3 ist,

wobei die einzelnen Gruppen Y gleich oder verschieden sein können, wenn m > 1 ist , dadurch gekennzeichnet, daß man Phenylpropargylether der Formel (II)

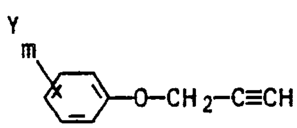

(II)

mit 2.2-Dimethylpropanal in Gegenwart einer Mineralbase in einem polar-aprotischen Lösungsmittel umsetzt.

2. Verfahren nach Anspruch 2, dadurch gekennzeichnet, daß man Kaliumhydroxide als Base verwendet.

3. Verfahren nach den Ansprüchen 2 oder 3, dadurch gekennzeichnet, daß man 0.5 bis 1 Äquivalente Kaliumhydroxid, bezogen auf (II), verwendet.

4. Verfahren nach den Ansprüchen 2 bis 4, dadurch gekennzeichnet, daß man als Lösungsmittel Ether verwendet.

Europäisches Patentamt

# EUROPÄISCHER RECHERCHENBERICHT

Nummer der Anmeldung

EP 88 12 1928

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 035 697 (BAYER)<br>* Ansprüche *<br>--- | 1 | C 07 C 43/23<br>C 07 C 41/30 //<br>C 07 C 49/255 |
| A | EP-A-0 040 350 (BASF)<br>* Ansprüche *<br>--- | 1 | |
| D,A | EP-A-0 150 404 (BASF)<br>* Ansprüche, Seite 3 *<br>--- | 1 | |
| A | CHEMICAL ABSTRACTS, Band 78, Nr. 11, 19. März 1973, Seite 426, Zusammenfassung Nr. 71594t, Columbus, Ohio, US; T. DZHURAKULOV et al.: "Synthesis of acetylenic alcohols made from propargyl ethers of substituted phenols", & IZV. VYSSH. UCHEB. ZAVED., KHIM. KHIM. TEKHNOL. 1972, 15(11), 1681-4<br>----- | 1-5 | |
| | | | **RECHERCHIERTE SACHGEBIETE (Int. Cl.4)** |
| | | | C 07 C 43/00 |

Der vorliegende Recherchenbericht wurde für alle Patentansprüche erstellt

| Recherchenort | Abschlußdatum der Recherche | Prüfer |
|---|---|---|
| DEN HAAG | 13-04-1989 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument